# EUROPEAN PATENT APPLICATION

(11) **EP 3 567 100 A1**
(43) Date of publication of application: **13.11.2019**
(21) Application number: 19181427.6
(22) Date of filing: 06.11.2015
(51) Int. Cl.: C12N 5/0735, C12N 5/00, C12N 5/10, A61K 35/12, A61K 35/545, A61P 1/16, A61P 1/18, A61P 7/00, A61P 9/00, A61P 13/12, A61P 19/00, A61P 25/00, A61P 27/02

(54) **DIFFERENTIATION-INDUCED CELL POPULATION FROM WHICH UNDIFFERENTIATED CELLS HAVE BEEN REMOVED, USE OF SAME, AND METHOD FOR PRODUCING SAME**

(30) Priority: 07.11.2014 JP 2014226682
(62) Divisional of application: 15856872.5
(71) Applicant: Osaka University, Suita-shi Osaka 5650871 (JP)
(72) Inventor: MASUDA, Shigeo, Osaka, 565-0871 (JP); SOUGAWA, Nagako, Osaka, 565-0871 (JP); FUKUSHIMA, Satsuki, Osaka, 565-0871 (JP); MIYAGAWA, Shigeru, Osaka, 565-0871 (JP); SAWA, Yoshiki, Osaka, 565-0871 (JP)
(74) Representative: Loyer & Abello

(57) **Abstract**

Provided is a cell population comprising differentiated cells obtainable by inducing differentiation of pluripotent stem cells, wherein the content ratio of undifferentiated pluripotent stem cells is 0.2% or less.

## Description

### Technical Field

The present invention relates to a differentiation-induced cell population from which undifferentiated cells have been removed, use of the same, and a method for producing the same.

### Background Art

Research and development have been actively conducted on cell populations comprising differentiated cells obtainable by inducing differentiation of pluripotent stem cells. The usefulness of such cell populations (pluripotent stem cell-processed products) as medicaments (cell pharmaceuticals), or as research tools for drug design, development, etc., has attracted attention. For use as medicaments, human cells are mainly used; whereas for use as research tools, not only human cells, but also cells derived from various organisms are used. Pluripotent stem cells, such iPS cells and ES cells, are widely used depending on the purpose.

However, in these differentiation-induced cell populations, pluripotent stem cells in an undifferentiated state inevitably remain and are incorporated. This is problematic because it is preferable that the purity of differentiation-induced cells is higher, whichever purpose is intended. In particular, when pluripotent stem cells, which have tumorigenicity in an undifferentiated state, are used, the use of differentiation-induced cell populations as medicaments involves a safety risk if the differentiation-induced cell populations contain undifferentiated pluripotent stem cells.

Therefore, various techniques for further improving the purity of differentiation-induced cell populations have been proposed. These techniques are roughly divided into two groups: a technique group that mainly improves differentiation induction efficiency, and a technique group that purifies and refines differentiation-induced cells. The latter group is further divided into two groups: a technique of selectively separating differentiation-induced cells (positive selection), and a technique of removing inevitably mixed undifferentiated pluripotent stem cells (negative selection). Techniques proposed to purify and refine differentiation-induced cells can be sorted by the difference in the approach as follows. For example, as an approach using special media, NPL 1 and NPL 2 propose methods that use methionine (-) media, and NPL 3 and NPL 4 propose methods that use sugarless media. Moreover, as an approach using survival signal inhibitors, NPL 5 proposes a method that uses a survivin inhibitor. Furthermore, as an approach using FACS sorting, NPL 6 proposes purging by an anti-SSEA-5 antibody, and NPL 7 proposes purging using a sugar chain antibody (lectin).

### Citation List

### Non-patent Literature

NPL 1: Matsuura K, Kodama F, Sugiyama K, Shimizu T, Hagiwara N, Okano T. Elimination of remaining undifferentiated iPS cells in the process of human cardiac cell sheet fabrication using a methionine-free culture condition. Tissue Eng Part C Methods. 2014 Sep 23. (Epub ahead of print)
NPL 2: Shiraki N, Shiraki Y, Tsuyama T, Obata F, Miura M, Nagae G, Aburatani H, Kume K, Endo F, Kume S. Methionine metabolism regulates maintenance and differentiation of human pluripotent stem cells. Cell Metab. 2014 May 6; 19(5): 780-94. NPL 3: Tohyama S, Hattori F, Sano M, Hishiki T, Nagahata Y, Matsuura T, Hashimoto H, Suzuki T, Yamashita H, Satoh Y, Egashira T, Seki T, Muraoka N, Yamakawa H, Ohgino Y, Tanaka T, Yoichi M, Yuasa S, Murata M, Suematsu M, Fukuda K. Distinct metabolic flow enables large-scale purification of mouse and human pluripotent stem cell-derived cardiomyocytes. Cell Stem Cell. 2013 Jan 3; 12(1): 127-37.
NPL 4: Hemmi N, Tohyama S, Nakajima K, Kanazawa H, Suzuki T, Hattori F, Seki T, Kishino Y, Hirano A, Okada M, Tabei R, Ohno R, Fujita C, Haruna T, Yuasa S, Sano M, Fujita J, Fukuda K. A Massive Suspension Culture System With Metabolic Purification for Human Pluripotent Stem Cell-Derived Cardiomyocytes. Stem Cells Transl Med. 2014 Oct 29. pii: sctm. 2014-0072. (Epub ahead of print)
NPL 5: Lee MO, Moon SH, Jeong HC, Yi JY, Lee TH, Shim SH, Rhee YH, Lee SH, Oh SJ, Lee MY, Han MJ, Cho YS, Chung HM, Kim KS, Cha HJ. Inhibition of pluripotent stem cell-derived teratoma formation by small molecules. Proc Natl Acad Sci USA. 2013 Aug 27; 110(35): E3281-90.
NPL 6: Tang C, Lee AS, Volkmer JP, Sahoo D, Nag D, Mosley AR, Inlay MA, Ardehali R, Chavez SL, Pera RR, Behr B, Wu JC, Weissman IL, Drukker M. An antibody against SSEA-5 glycan on human pluripotent stem cells enables removal of teratoma-forming cells. Nat Biotechnol. 2011 Aug 14; 29(9): 829-34.
NPL 7: Onuma Y, Tateno H, Hirabayashi J, Ito Y, Asashima M. rBC2LCN, a new probe for live cell imaging of human pluripotent stem cells. Biochem Biophys Res Commun. 2013 Feb 15; 431(3): 524-9.

### Summary of Invention

### Technical Problem

An object of the present invention is to provide a cell population comprising differentiated cells obtainable by inducing differentiation of pluripotent stem cells, wherein the content ratio of undifferentiated pluripotent stem cells is lower than before.

### Solution to Problem

The present inventors succeeded in obtaining, by several novel methods, a cell population comprising differentiated cells obtainable by inducing differentiation of pluripotent stem cells, wherein the content ratio of undifferentiated pluripotent stem cells is 0.2% or less. The present invention includes the following embodiments.
Item 1-1. A cell population comprising differentiated cells obtainable by inducing differentiation of pluripotent stem cells, wherein the content ratio of undifferentiated pluripotent stem cells is 0.2% or less.
Item 1-2. The cell population according to Item 1-1, wherein the pluripotent stem cells are induced pluripotent stem cells or embryonic stem cells.
Item 1-3. The cell population according to Item 1-2, wherein the induced pluripotent stem cells are iPS cells.
Item 1-4. The cell population according to Item 1-2, wherein the embryonic stem cells are ES cells.
Item 1-5. The cell population according to any one of Items 1-1 to 1-4, wherein the total number of cells is 1 x 10⁴ or more.
Item 1-6. The cell population according to any one of Items 1-1 to 1-5, wherein the content ratio of undifferentiated pluripotent stem cells is determined by undifferentiated cell marker analysis using flow cytometry.
Item 1-7. The cell population according to any one of Items 1-1 to 1-6 for use as a medicament.
Item 2-1. A method for producing a cell population comprising differentiated cells obtainable by inducing differentiation of pluripotent stem cells, the method comprising:
   (A) culturing, in the presence of an anti-CD30 antibody-binding agent, a cell population comprising differentiated cells obtainable by inducing differentiation of pluripotent stem cells, thereby reducing the content ratio of undifferentiated pluripotent stem cells; and/or
   (B) culturing, in the presence of a BET inhibitor, a cell population comprising differentiated cells obtainable by inducing differentiation of pluripotent stem cells, thereby reducing the content ratio of undifferentiated pluripotent stem cells.
Item 2-2. The production method according to Item 2-1, wherein the pluripotent stem cells are induced pluripotent stem cells or embryonic stem cells.
Item 2-3. The production method according to Item 2-2, wherein the induced pluripotent stem cells are iPS cells.
Item 2-4. The production method according to Item 2-2, wherein the embryonic stem cells are ES cells.
Item 3-1. A composition comprising an anti-CD30 antibody-binding agent for use in reducing the content ratio of undifferentiated pluripotent stem cells in a method for producing a cell population comprising differentiated cells obtainable by inducing differentiation of pluripotent stem cells.
Item 3-2. A composition comprising a BET inhibitor, the composition for use in reducing the content ratio of undifferentiated pluripotent stem cells in a method for producing a cell population comprising differentiated cells obtainable by inducing differentiation of pluripotent stem cells.

### Advantageous Effects of Invention

The present invention allows use of differentiation-induced cell populations with a purity higher than that of conventional cell populations, and provides, for example, medicaments with far fewer side effects, such as tumorigenicity, and research tools of higher accuracy.

### Brief Description of Drawings

- Fig. 1: shows the results of Example 1.
- Fig. 2: shows the results of Example 1.
- Fig. 3: shows the results of Example 2.
- Fig. 4: shows the results of Example 2.
- Fig. 5: shows the results of Example 2.
- Fig. 6: shows the results of Example 3.
- Fig. 7: shows the results of Example 4.
- Fig. 8: shows the results of Example 4.
- Fig. 9: shows the results of Example 5.
- Fig. 10: shows the results of Example 6.
- Fig. 11: shows the results of the Test Example.
- Fig. 12: shows the results of the Test Example.
- Fig. 13: shows the results of Example 7.

### Description of Embodiments

### 1. Cell population of the present invention

The present invention relates to a cell population comprising differentiated cells obtainable by inducing differentiation of pluripotent stem cells, wherein the content ratio of undifferentiated pluripotent stem cells is 0.2% or less.

In the cell population of the present invention, the content ratio of undifferentiated pluripotent stem cells is preferably 0.1% or less, and more preferably 0.05% or less.

The pluripotent stem cells are not particularly limited, and a wide range of pluripotent stem cells can be used. The pluripotent stem cells may be induced pluripotent stem cells or embryonic stem cells.

The induced pluripotent stem cells are not particularly limited. For example, iPS cells and the like can be used. The embryonic stem cells are not particularly limited. For example, ES cells and the like can be used. Among these, iPS cells and ES cells are particularly preferable in terms of safety etc., particularly for use as medicaments.

The present invention is characterized in that the content ratio (i.e., residual ratio) of undifferentiated pluripotent stem cells in the cell population is lower than before. That is, the present invention has been completed based on the development of novel methods that can realize a conventionally unrealizable residual ratio of undifferentiated pluripotent stem cells. Specifically, these novel methods are techniques of performing purification while focusing on the characteristics of pluripotent stem cells remaining in an undifferentiated state, as described later. Therefore, in the present invention, the type of differentiated cell is not particularly limited.

Examples of differentiated cells include, but are not limited to, cardiomyocytes, nerve cells, retinal cells (e.g., retinal pigment epithelial cells), blood (hematopoietic) cells, liver cells, pancreatic beta cells, renal cells, cartilage cells, reproductive cells, and the like. Particularly preferable among these are cardiomyocytes.

The cell population of the present invention is characterized in that the content ratio of undifferentiated pluripotent stem cells is low. The cell population of the present invention may further contain cells different from differentiation-induced cells and undifferentiated pluripotent stem cells, as required.

The total number of cells in the cell population of the present invention is not particularly limited. The total number of cells is generally 1 x 10⁴ or more, preferably 1 x 10⁵ to 1 x 10⁹, and more preferably 1 x 10⁸ to 1 x 10⁹.

The content ratio of undifferentiated pluripotent stem cells in the cell population of the present invention can be defined as one obtained by undifferentiated cell marker analysis using flow cytometry. Specifically, this analysis can be conducted in the following manner.

After differentiation of undifferentiated pluripotent stem cells is induced, a cell dissociation reagent, such as Accutase, is applied to about 1 x 10⁵ to 5 x 10⁵ differentiated cells at 37°C for 5 minutes, and differentiated cells are dissociated and collected by centrifugation. The differentiated cells are suspended in 100 µ of PBS, and an anti-human TRA-1-60 antibody and an isotype control antibody, both of which are fluorescently labeled, are each added. Then, the resultants are each reacted at 4°C for 20 minutes in a dark place. After the differentiated cells are washed with PBS and collected by centrifugation, they are each suspended in 300 µ of PBS, passed through a cell strainer, and then collected in FACS tubes. In flow cytometry, fluorescent emission is detected by a detector corresponding to labeled fluorescence, and quantified. Fractions stained with the isotype control antibody are defined as negative fractions, whereas fractions with a higher fluorescence intensity are defined as positive fractions. Among fractions stained with the anti-human TRA-1-60 antibody, the ratio belonging to the positive fractions is expressed as a percentage.

The cell population of the present invention can be used for various purposes, without limitation. For example, because the content ratio of undifferentiated pluripotent stem cells is lower, use of the cell population of the present invention as a medicament (cell pharmaceutical) involves less safety risk, and is preferable. Use as a medicament, that is, the target disease, dosage, and usage, can be suitably determined depending on the type of differentiated cell. Although it is not particularly limited, for example, when the differentiated cells are cardiomyocytes, the cell population of the present invention can be used for the purpose of treating serious heart failure etc. involved in myocardial infarction and cardiomyopathy (e.g., dilated cardiomyopathy).

When used as a medicament, the cell population of the present invention has the effect such that safety risk, such as tumorigenicity, is reduced.

When used as a medicament, the cell population of the present invention can be used as an active ingredient of a pharmaceutical composition. Such a pharmaceutical composition is not particularly limited, and may contain various other components that can be contained in so-called cell pharmaceuticals.

In the cell population of the present invention, the cell population explained above may be contained in a cell culture medium, or formed into a sheet, depending on the purpose of use etc.

### 2. Method for producing the cell population of the present invention

The cell population of the present invention can be obtained by the methods explained below, although it is not particularly limited thereto. These methods can be combined, if necessary. These methods both belong to the technique of removing inevitably mixed undifferentiated pluripotent stem cells, and are novel methods that can reduce the content ratio of undifferentiated pluripotent stem cells to a level that could not be achieved by conventional methods.

### (1) Method using an anti-CD30 antibody-binding agent

This method uses an anti-CD30 antibody-binding agent comprising an antibody that specifically recognizes CD30, which is known as an undifferentiated cell marker.

CD30 belongs to the TNF-R superfamily, and is expressed on some lymphomas. In normal tissue, CD30 is supposed to be expressed only on activated lymphocytes. NF-KB and MAPK pathway are known as downstream signals. CD30 is considered to contribute to survival through these signals.

Specifically, the anti-CD30 antibody-binding agent is an antibody-drug conjugate (ADC) in which a drug having cell-killing activity is bound by a linker cleavable in the intracellular environment. The anti-CD30 antibody-binding agent enters cells recognized by the antibody, and is released into the cells, thereby selectively killing the cells.

The agent is not particularly limited. Examples include microtubule inhibitor MMAE and the like.

Further, the linker is not particularly limited. Examples include a linker that can be cleaved by proteolytic enzymes, and other linkers.

Examples of the anti-CD30 antibody-binding agent include, but are not limited to, Adcetris (registered trademark) (SGN-35) (Millennium, Seattle Genetics, and Takeda Chemical), which is commercially available as an antibody drug. Adcetris (registered trademark) was approved by the FDA in 2011 as being applicable to CD30-positive lymphoma. In Japan, Adcetris (registered trademark) was also approved by the Pharmaceutical Affairs in April 2014.

The content ratio of undifferentiated pluripotent stem cells can be reduced by culturing a cell population comprising differentiated cells obtainable by inducing differentiation of pluripotent stem cells in the presence of an anti-CD30 antibody-binding agent.

The concentration of the anti-CD30 antibody-binding agent in the cell medium can be suitably determined depending on the type of pluripotent stem cell etc. The concentration of the anti-CD30 antibody-binding agent is preferably 5 µg/ml to 50 µg/ml, more preferably 10 µg/ml to 50 µg/ml, and even more preferably 25 µg/ml to 50 µg/ml.

In the above, the culture time can also be suitably determined depending on the type of pluripotent stem cell etc. The culture time is preferably 24 hours to 96 hours, more preferably 48 hours to 96 hours, and even more preferably 72 hours to 96 hours.

### (2) Method using a BET inhibitor

This method uses a BET inhibitor, which is a histone acetylation inhibitor.

The BET (bromodomain and extra terminal) protein family consists of BRD2, BRD3, BRD4, and BRDT, and is deeply involved in transcriptional regulation by RNA polymerase II. The BET protein family recognizes acetylated lysine residues of histone tails via the bromodomain (epigenetic reader), and recruits transcription regulatory complexes to acetylated chromatin. BET inhibitors, such as low-molecular-weight compound JQ1, are reported to have a therapeutic role in cancer and inflammation (Nature. 2010; 468: pp. 1067-73 and Cell. 2011; 146: pp. 904-17). In particular, BRD4 is considered to regulate the expression of c-Myc, NK-KB, and Nanog in several types of cancer. This is attributable to BRD4-binding to super-enhancers.

Undifferentiated pluripotent stem cells are killed by culturing them in the presence of a BET inhibitor. As in the above method (1), the content ratio of undifferentiated pluripotent stem cells can be reduced by culturing a cell population comprising differentiated cells obtainable by inducing differentiation of pluripotent stem cells in the presence of a BET inhibitor.

Examples of the BET inhibitor include I-BET151, I-BET762, etc., in addition to JQ1.

The concentration of the BET inhibitor in the cell medium can be suitably determined depending on the type of BET inhibitor, the type of pluripotent stem cell, etc. For example, when JQ1 is used, the concentration thereof is preferably 0.1 µM to 10 µM, more preferably 0.2 µM to 10 µM, and even more preferably 0.5 µM to 10 µM.

In the above, the culture time can also be suitably determined depending on the type of BET inhibitor, the type of pluripotent stem cell, etc. For example, when JQ1 is used, the culture time is preferably 24 hours to 96 hours, more preferably 48 hours to 96 hours, and even more preferably 72 hours to 96 hours.

### 3. Composition of the present invention

The present invention further provides the following compositions that are used in the above methods.
(a) A composition comprising an anti-CD30 antibody-binding agent;
(b) a composition comprising a BET inhibitor; and
(c) a composition comprising an anti-CD30 antibody-binding agent and a BET inhibitor.

The mixing concentration of the anti-CD30 antibody-binding agent and/or the BET inhibitor in these compositions is not particularly limited, and can be suitably determined depending on the required storage stability, purpose of use, etc.

These compositions may further contain other components, as long as the above methods are not disturbed. Examples of other components include, but are not limited to, sugars, such as glucose, maltose, sucrose, lactose, raffinose, trehalose, mannitol, hydroxyethyl starch, and pullulan; organic acids, such as gluconic acid, lactic acid, acetic acid, propionic acid, β-hydroxybutyric acid, and citric acid; electrolytes, such as sodium chloride, potassium chloride, magnesium chloride, calcium chloride, sodium dihydrogen phosphate, potassium dihydrogen phosphate, disodium hydrogen phosphate, dipotassium hydrogen phosphate, sodium hydrogen carbonate, potassium hydrogen carbonate, sodium carbonate, and potassium carbonate; vitamins, such as L-ascorbic acid and vitamin E; amino acids, such as glycine, glutamic acid, and lysine; hormones, such as antidiuretic hormone and insulin; anticoagulants, such as citric acid, citrate, heparin, and disodium edetate, antihypertensive agents, such as calcium channel blockers, adrenergic β-receptor antagonists, and angiotensin-converting enzyme inhibitors; nucleobases, such as adenosine triphosphate; anti-freezing agents, such as antifreeze protein; as well as active oxygen eliminators, cell activators, antibiotics, antiplatelet factors, hepatopathy inhibitors, excipients, binders, disintegrators, dispersants, viscous agents, reabsorption promoters, surfactants, solubilizers, preservatives, antiseptics, emulsifiers, isotonizing agents, stabilizers, buffers, pH-regulators, and the like. At least one of these components can be mixed, as necessary.

### Examples

The present invention is described in more detail below with reference to Examples. The present invention is not limited to the following Examples.

### Example 1: Method for removing undifferentiated cells using an anti-CD30 antibody-binding agent

Human iPS cell strain MYH-GIP4 was seeded in a 12-well plate (cell density: 20% confluency). Forty-eight hours after seeding, 0, 0.05, and 5 µg/ml of an anti-CD30 antibody-binding agent (general name: Brentuximab vedotin, abbreviated as "BV") (trade name: Adcetris (registered trademark)) was added to each well (the agent-containing medium was exchanged every 24 hours).

Thereafter, microscope observation was performed every 24 hours. Since Adcetris (registered trademark) contains additives, in addition to BV, numerical values converted from the net weight of BV were described (i.e., "0, 0.05, and 5 µg/ml as BV").

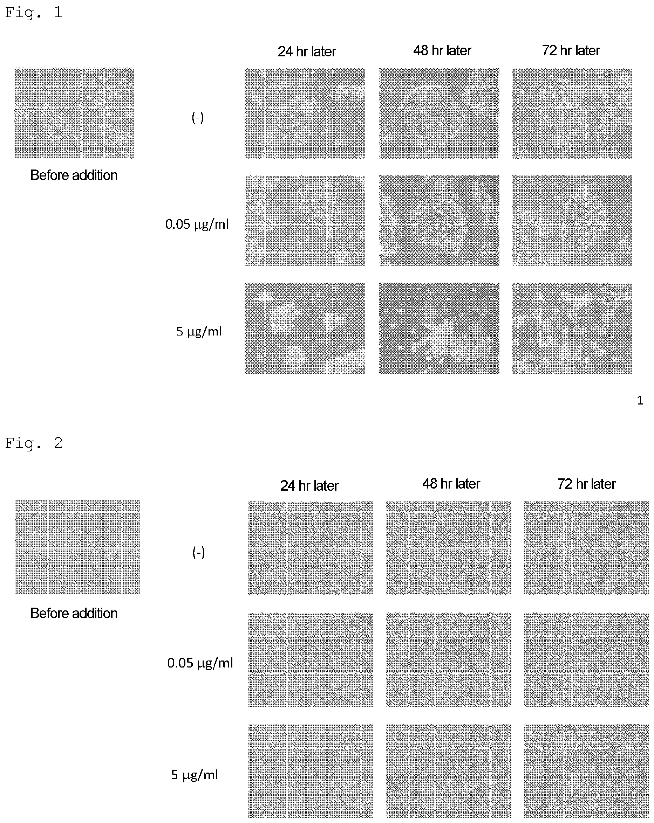

The results confirmed that cell death was partially induced in the 5-µg/ml BV-added group 48 to 72 hours later (Fig. 1) .

Similarly, in order to examine the influence of BV on normal human dermal fibroblasts (NHDF), the same experiment as in Example 1 was conducted.

Human fibroblasts (NHDF) were seeded in a 12-well plate (cell density: 50% confluency). Forty-eight hours after seeding, 0, 0.05, and 5 µg/ml of BV was added to each well (the agent-containing medium was exchanged every 24 hours). Thereafter, microscope observation was performed every 24 hours.

The results show that induction of cell death was not observed macroscopically 72 hours after the addition of 5 µg/ml BV (Fig. 2).

### Example 2: Method for removing undifferentiated cells using an anti-CD30 antibody-binding agent

In order to clarify how the number of human iPS cells changed with various BV concentrations and BV treatment times, BV was added *in vitro* to human iPS cells, cell proliferation assay (CCK-8 assay), which reflects the number of cells, was performed, and viable cells were evaluated quantitatively.

Three human iPS cell strains (1231A3, MYH-GIP4, and 253G1) and NHDF were each seeded in a 12-well plate (cell density: 20% confluency). However, the cell density of NHDF was set to 50% confluency.

Forty-eight hours after seeding, 0, 5, 25, and 50 µg/ml of BV was added to each well (the agent-containing medium was exchanged every 24 hours). Thereafter, cell proliferation assay was performed 24 hours, 48 hours, and 72 hours after addition.

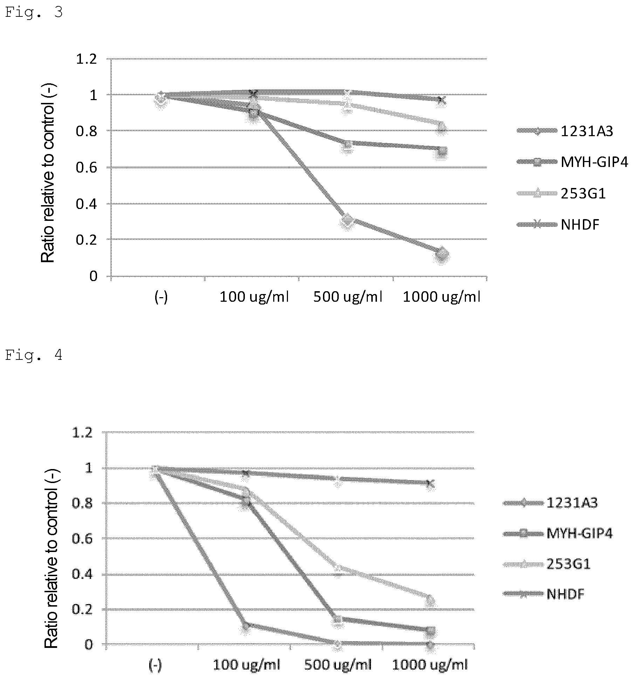

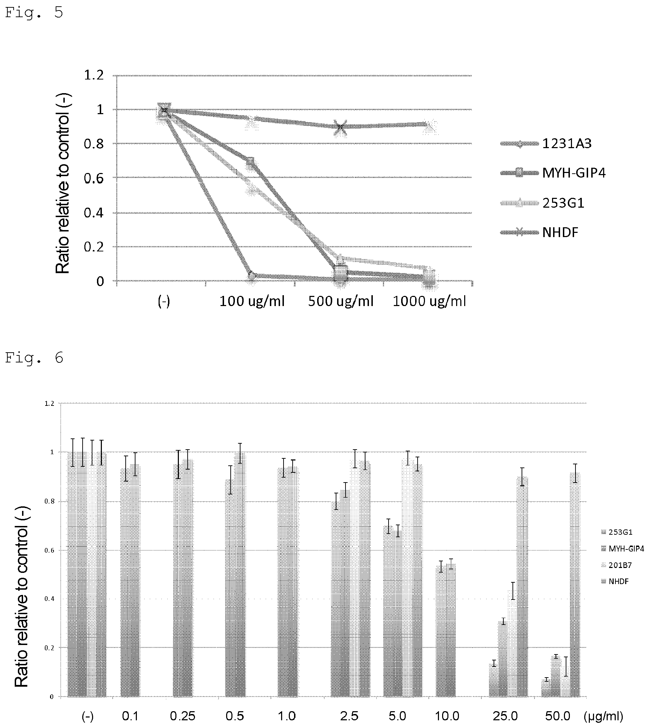

Fig. 3 (24 hours later), Fig. 4 (48 hours later), and Fig. 5 (72 hours later) show the results. In the human iPS cells, the highest cytostatic effect was observed 72 hours later, and the concentration-dependent effects of BV were also confirmed. The BV concentration at which the number of cells was almost completely suppressed for 72 hours was 5 to 50 µg/ml for 1231A3, and 25 to 50 µg/ml for MYH-GIP4 and 253G1. On the other hand, it was confirmed that NHDF was hardly affected by BV.

### Example 3: Method for removing undifferentiated cells using an anti-CD30 antibody-binding agent

In order to clarify how the number of human iPS cells changed with various BV concentrations and BV treatment times, BV was added *in vitro* to human iPS cells, cell proliferation assay (CCK-8 assay), which reflects the number of cells, was performed, and viable cells were evaluated quantitatively.

Three human iPS cell strains (253G1, MYH-GIP4, and 201B7) and NHDF were each seeded in a 12-well plate (cell density: 20% confluency). However, the cell density of NHDF was set to 50% confluency.

Forty-eight hours after seeding, 0, 0.1, 0.25, 0.5, 1, 2.5, 5, 10, 25, and 50 µg/ml of BV was added to each well (the agent-containing medium was exchanged every 24 hours). Thereafter, cell proliferation assay was performed 72 hours after addition.

Fig. 6 shows the results. The concentration-dependent effects of BV were confirmed in the human iPS cells. In particular, it was revealed that the cell proliferation of all the human iPS cells was suppressed to 20% or less 72 hours after treatment with 50 µg/ml BV. On the other hand, it was confirmed that NHDF was hardly affected by BV.

### Example 4: Method for removing undifferentiated cells using an anti-CD30 antibody-binding agent

This method is targeted for human iPS cell-derived cardiomyocytes (iPS-CM). iPS-CM is considered to contain tumorigenic undifferentiated cells, in addition to cardiomyocytes.

The method for inducing differentiation into cardiomyocytes was performed according to the following document: Matsuura K, et al. Creation of human cardiac cell sheets using pluripotent stem cells. Biochem Biophys Res Commun, 2012, and 425(2) pp. 321-7. iPS-CM derived from human iPS cell strain 253G1 was seeded in a 12-well plate (cell density: 90% confluency). Forty-eight hours after seeding, 0, 5, and 10 µg/ml of BV was added to each well (the agent-containing medium was exchanged every 24 hours) . Thereafter, mRNA was extracted 72 hours and 96 hours after addition. Lin28 expression levels were quantified by quantitative PCR.

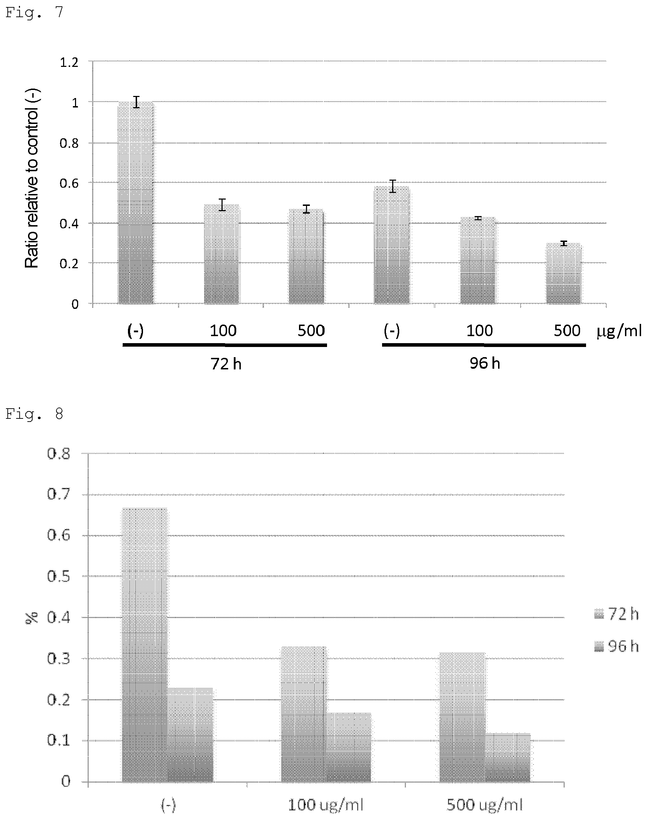

The results show that in the 72-hour treatment groups, the expression of undifferentiated marker Lin28 in the BV-added groups was reduced to about 50% of the control group (BV: 0 µg/ml). In the 96-hour treatment groups, the expression of Lin28 in the BV-added groups was reduced to about 50 to 70% of the control group (Fig. 7).

Moreover, when the Lin28 expression level of pure human iPS cells was regarded as 100%, the Lin28 expression level of the control group at the 72nd hour was about 0.7%, whereas the Lin28 expression level of the BV-added groups at the 72nd hour was reduced to about 0.3%. Further, the Lin28 expression level of the BV-added groups at the 96th hour was reduced to about 0.1% (Fig. 8) .

The above results demonstrated that undifferentiated cells can be efficiently removed by treating iPS-CM with BV. In particular, when undifferentiated marker Lin28 was used as an index, the Lin28 positive rate in iPS-CM was suppressed to 0.1% after BV treatment, compared with human iPS cells. This was considered to be attributable to the powerful undifferentiated cell-removing effect of BV.

### Example 5: Method for removing undifferentiated cells using a BET inhibitor

In order to examine the influence of a BET inhibitor (name of the drug used: JQ1) on human iPS cells and normal human dermal fibroblasts (NHDF), JQ1 was added *in vitro* to human iPS cells and normal human dermal fibroblasts (NHDF), and cell proliferation and cell death were observed macroscopically.

Human iPS cell strain 253G1 was seeded in a 12-well plate (cell density: 20% confluency). Human fibroblasts (NHDF) were seeded in a 12-well plate (cell density: 50% confluency).

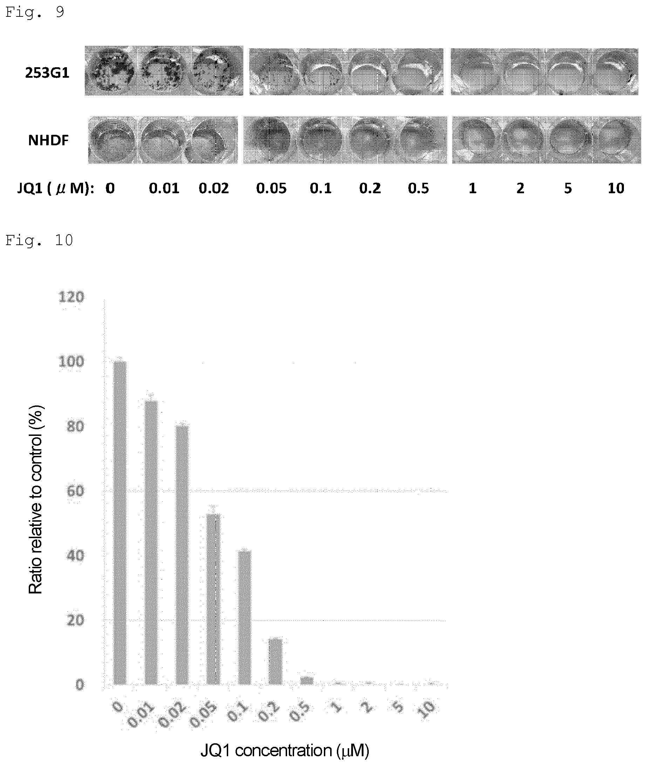

Forty-eight hours after seeding, 0, 0.01, 0.02, 0.05, 0.1, 0.2, 0.5, 1, 2, 5, and 10 µM of JQ1 was added to each well (the agent-containing medium was exchanged every 48 hours). Thereafter, microscope observation was performed 96 hours later, and crystal violet staining was carried out to visualize the cells (Fig. 9).

The results confirmed that regarding the human iPS cells, complete cell death was induced 96 hours later in the groups to which 0.2 µM or more of JQ1 was added. On the other hand, cell death was not observed in NHDF.

### Example 6: Method for removing undifferentiated cells using a BET inhibitor

In order to clarify how the number of human iPS cells changed with various JQ1 concentrations, JQ1 was added *in vitro* to human iPS cells, cell proliferation assay (CCK-8 assay), which reflects the number of cells, was performed 96 hours later, and viable cells were evaluated quantitatively.

Human iPS cell strain 253G1 was seeded in a 12-well plate (cell density: 20% confluency).

Forty-eight hours after seeding, 0, 0.01, 0.02, 0.05, 0.1, 0.2, 0.5, 1, 2, 5, and 10 µM of JQ1 was added to each well (the agent-containing medium was exchanged every 48 hours). Thereafter, cell proliferation assay was performed 96 hours after addition (Fig. 10).

The results confirmed that the growth of the Human iPS cell 253G1 was suppressed to a cell proliferation rate of 10% or less 96 hours later in the groups to which 0.2 µM or more of JQ1 was added.

### Test Example: JQ1 toxicity test

In order to determine that JQ1 was not toxic against human normal differentiated cells (NHDF and iPS-CM), the same cell proliferation assay as in Example 6 was conducted.

NHDF and iPS-CM were each seeded in a 12-well plate (cell density: 50% and 90% confluency, respectively).

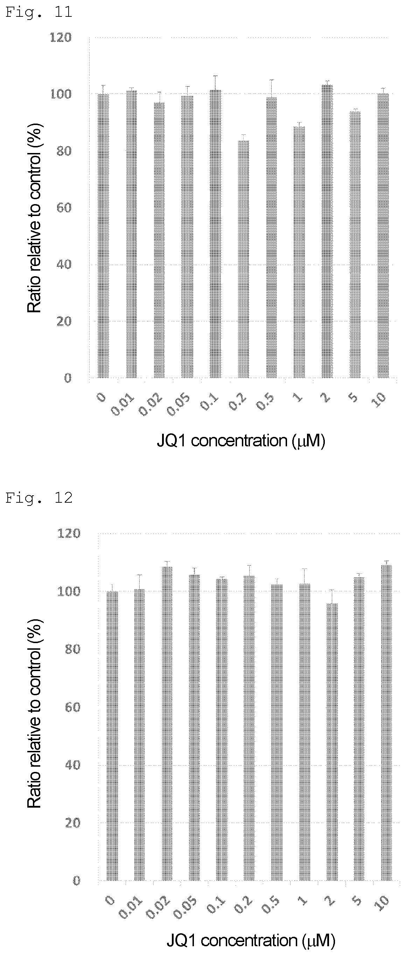

Figs. 11 and 12 show the results of NHDF and iPS-CM, respectively. It was confirmed that JQ1 was almost not toxic against NHDF and iPS-CM.

### Example 7: Method for removing undifferentiated cells using a BET inhibitor

iPS-CM derived from human iPS cell strain MYH-GIP4 was seeded in a 12-well plate (cell density: 90% confluency). Forty-eight hours after seeding, 0, 1, and 5 µMl of JQ1 was added to each well (the agent-containing medium was exchanged every 48 hours). Thereafter, the TRA-1-60 positive rate was quantified 96 hours after addition. As a control, the same measurement was performed using an isotype control antibody, and it was confirmed that there were no false-positives.

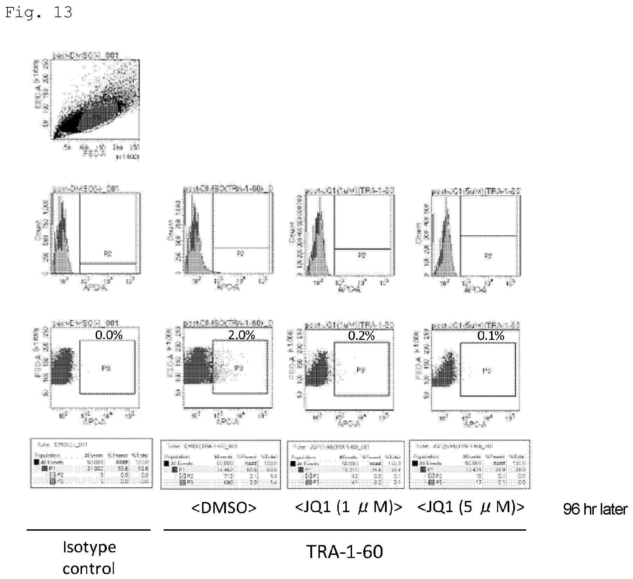

The results (Fig. 13) show that the TRA-1-60 positive rate was 2.0% in the control (JQ1: 0 µM) group, whereas the positive rate was reduced to 0.2% in the 1-µM JQ1-added group, and to 0.1% in the 5-µM JQ1-added group.

The above results demonstrated that undifferentiated cells can be efficiently removed by treating iPS-CM with JQ1. In particular, when undifferentiated marker TRA-1-60 was used as an index, the TRA-1-60 positive rate in iPS-CM was suppressed to 0.1% after JQ1 treatment, as compared with human iPS cells. This was considered to be attributable to the powerful undifferentiated cell-removing effect of JQ1.

## Claims

1. A cell population comprising differentiated cells obtainable by inducing differentiation of pluripotent stem cells, wherein the content ratio of undifferentiated pluripotent stem cells is 0.2% or less.

2. The cell population according to claim 1, wherein the pluripotent stem cells are induced pluripotent stem cells or embryonic stem cells.

3. The cell population according to claim 2, wherein the induced pluripotent stem cells are iPS cells.

4. The cell population according to claim 2, wherein the embryonic stem cells are ES cells.

5. The cell population according to any one of claims 1 to 4, wherein the total number of cells is 1 x 10⁴ or more.

6. The cell population according to any one of claims 1 to 5, wherein the content ratio of undifferentiated pluripotent stem cells is determined by undifferentiated cell marker analysis using flow cytometry.

7. The cell population according to any one of claims 1 to 6 for use as a medicament.
